# EUROPÄISCHE PATENTSCHRIFT

(11) **EP 1 569 555 B1**
(45) Veröffentlichungstag und Bekanntmachung des Hinweises auf die Patenterteilung: **10.09.2008**
(21) Anmeldenummer: 03811746.1
(22) Anmeldetag: 31.10.2003
(51) Int. Cl.: A61B 6/00, H05G 1/02

(54) **RÖNTGENSTATIV**
X-RAY STAND
STATIF DE RADIOGRAPHIE

(30) Priorität: 28.11.2002 SE 0203517
(43) Veröffentlichungstag der Anmeldung: 07.09.2005
(73) Patentinhaber: SIEMENS AKTIENGESELLSCHAFT, 80333 München (DE)
(72) Erfinder: ENGSTRÖM, Göran, S-17153 Johanneshov (SE); LUNDBERG, Mikael, S-19435 Upplands Väsby (SE); MELLSTRÖM, Erik, S-17565 Järfälla (SE)
(86) Internationale Anmeldenummer: PCT/EP2003/012156
(87) Internationale Veröffentlichungsnummer: WO 2004/047644

(56) Entgegenhaltungen:
- US-A- 5 410 584
- US-B1- 6 428 206

## Beschreibung

Die Erfindung betrifft ein Röntgenstativ mit einem äußeren C-Bogen, entlang welchem eine Halterung für einen inneren C-Bogen verschiebbar angeordnet ist, wobei der innere C-Bogen, der eine Röntgenröhre und einen Bildverstärker trägt, in der Halterung verschiebbar angeordnet ist sowie eine Antriebsvorrichtung für die Verschiebung der Halterung entlang dem äußeren C-Bogen und für die Verschiebung des inneren C-Bogens entlang der Halterung.

Ein Röntgenstativ der obengenannten Art ist in der US-PS 5,410,584 gezeigt und beschrieben. Die Antriebsvorrichtung dieses Röntgenstativs umfasst zwei Motoren, wobei der eine Motor dazu vorgesehen ist, die Halterung entlang dem äußeren C-Bogen zu verschieben und der andere Motor ist dazu vorgesehen, den inneren C-Bogen entlang der Halterung zu verschieben. Mit einem Röntgenstativ dieser Art können orbitale Verschiebungen von etwa ± 100° erfolgen. Im Zusammenhang mit solchen großen orbitalen Verschiebungen kann es mit einer Antriebsvorrichtung, bestehend aus mehreren Motoren schwer sein, ein gleichmäßiges Fahren des inneren C-Bogens und eine gewünschte Genauigkeit der Positionierung, ohne eine regeltechnische komplizierte Lösung zu erreichen. Eine Antriebsvorrichtung dieser Art ist daher häufig teuer und erfordert außerdem viel Raum.

Aus der US 6,428,206 ist eine Röntgendiagnostikeinrichtung bekannt, bei der der Röntgenstrahler und der Röntgendetektor an einem halbkreisförmigen C-Bogen gehalten werden. Der Röntgengenerator ist mittels eines ersten Linearmotors mit einem ersten viertelkreisförmigen Arm verbunden, der seinerseits mit einem zweiten Arm durch einen zweiten Linearmotor bewegbar gehalten ist. Mittels eines dritten Motors ist dieser zweite Arm unabhängig von den anderen Motoren bewegbar. Der Halterungssockel ist auf dem Boden, an einer Wand oder an der Decke montiert. Auch hier weist die Röntgendiagnostikeinrichtung zur orbitalen Verschiebung mehrere Motore auf, die zur genauen Positionierung, insbesondere bei der in der US-PS 6,428,206 beschriebenen Aufnahmetechnik, aus einer Vielzahl von möglichen Positionen eine sehr genaue und synchronisierte Steuerung erfordert.

Der Erfindung liegt die Aufgabe zugrunde, ein Röntgenstativ der eingangs genannten Art zu schaffen mit einer Antriebsvorrichtung, die verhältnismäßig billig ist, die wenig Raum benötigt und mit deren Hilfe eine verhältnismäßig hohe Positionierungsgenauigkeit erreicht werden kann.

Diese Aufgabe wird erfindungsgemäß dadurch gelöst, das die Antriebsvorrichtung aus einem einzigen Antriebsmittel besteht, das den inneren C-Bogen und die Halterung derart gleichzeitig beeinflusst, dass sich der innere C-Bogen und die Halterung in dieselbe Richtung bewegen, und die Antriebsvorrichtung den inneren C-Bogen und die Halterung mit jeweils einer Übersetzung beeinflusst, wobei die Übersetzungen sich gegenseitig derart verhalten wie die Längen der beiden C-Bögen. Das Antriebsmittel ist vorzugsweise ein Motor. Dadurch, dass lediglich ein Motor gesteuert werden soll, ist der Antrieb im Aufbau einfach und daher billig. Außerdem ist es leicht zu regulieren. Die Genauigkeit der Positionierung kann nun mit einer verhältnismäßig einfachen regeltechnischen Anordnung groß gemacht werden. Der Raum für die Antriebsvorrichtung kann aufgrund des einfachen Aufbaus der Vorrichtung verhältnismäßig klein gehalten werden. Abhängig von der Wahl der Übersetzungen können die Längen des äußeren und des inneren C-Bogens gewählt werden, damit eine gewünschte optimale Länge der Teleskopbewegung der C-Bögen bei einem Röntgenstativ der genannten Art erhalten wird.

Die Antriebsvorrichtung nach der Erfindung ist vorzugsweise in der Halterung angeordnet.

In einer verhältnismäßig einfachen Ausführungsform der Antriebsvorrichtung nach der Erfindung wird vorgeschlagen, dass die Übersetzung über einen Zahnradantrieb erfolgt.

In einer vorteilhaften Weiterentwicklung der Antriebsvorrichtung nach der Erfindung wird vorgeschlagen, dass das Antriebsmittel mit Hilfe einer ersten Riementransmission den inneren C-Bogen verschiebt sowie über eine Kupplungstransmission ein Kupplungsrad antreibt, das seinerseits über eine zweite Riementransmission die Halterung verschiebt, wobei die Enden des Riemens der ersten Riementransmission an dem inneren C-Bogen und die Enden des Riemens der zweiten Riementransmission an dem äußeren C-Bogen befestigt sind.

Dieser Aufbau der Antriebsvorrichtung gewährleistet eine verhältnismäßig vibrationsfreie und damit eine gleichmäßige C-Bogenbewegung auch in Verbindung mit schnellen C-Bogenbewegungen und langen Bewegungsstrecken.

Die Kupplungstransmission zwischen der ersten und der zweiten Riementransmission kann ein Antriebsriemen oder eine Rollenkette sein. Die Kupplungstransmission kann auch nach der Erfindung eine Zahnradtransmission sein.

Da die Antriebsvorrichtung in der Halterung angeordnet ist, ist der äußere C-Bogen vorzugsweise über eine Welle mit einem Stativarm drehbar verbunden. Somit kann auch der innere C-Bogen mit der Halterung um die erwähnte Welle gedreht werden.

Die Erfindung ist nachfolgend anhand eines in den Zeichnungen dargestellten Ausführungsbeispiels näher erläutert. Es zeigen:
- FIG. 1: eine Seitenansicht eines Röntgenstativs mit einem derartigen Aufbau, dass eine Antriebsvorrichtung nach der Erfindung benutzt werden kann,
- FIG. 2: einen schematischen Aufbau einer Antriebsvorrich- tung nach der Erfindung,
- FIG. 3: einen schematischen Aufbau einer zweiten Antriebs- vorrichtung nach der Erfindung und
- FIG. 4: einen schematischen Aufbau einer dritten Antriebs- vorrichtung nach der Erfindung.

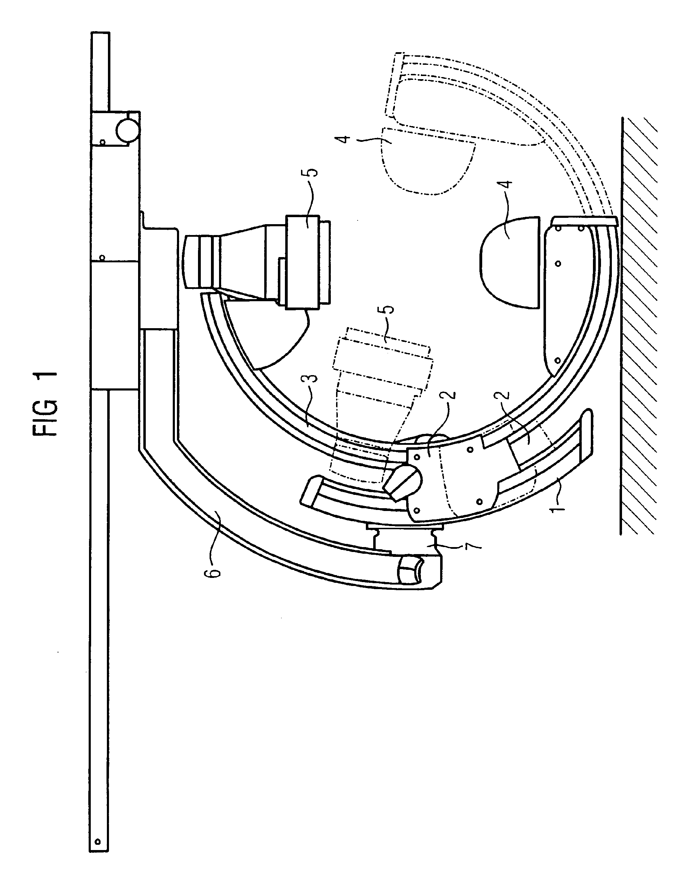

In der FIG. 1 ist ein an der Decke aufgehängtes Röntgenstativ gezeigt mit einem äußeren C-Bogen 1, entlang welchem eine Halterung 2 für einen inneren C-Bogen 3 verschiebbar angeordnet ist. Der innere C-Bogen 3, der eine Röntgenröhre 4 und einen Bildverstärker 5 trägt, ist in der Halterung 2 verschiebbar angeordnet. Eine in der Figur nicht gezeigte, später näher beschriebene Antriebsvorrichtung für die Verschiebung der Halterung 2 entlang dem äußeren C-Bogen 1 und für die Verschiebung des inneren C-Bogens 3 entlang der Halterung 2 ist in der Halterung 2 angeordnet. Der äußere C-Bogen 1 und damit auch die Halterung 2 und der innere C-Bogen 3 sind über eine Welle 7 mit einem Stativarm 6 des Stativs drehbar verbunden.

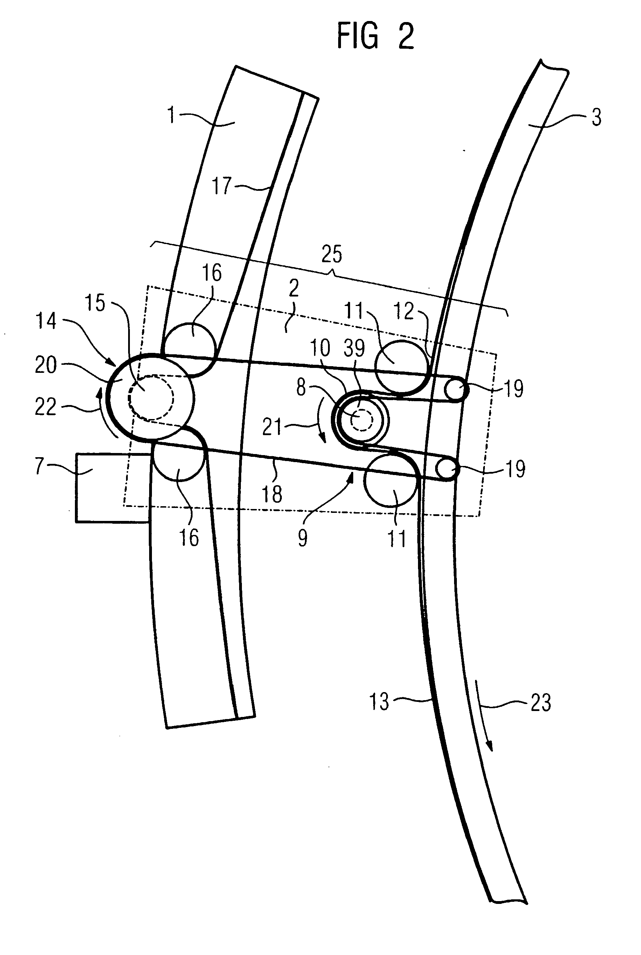

In der Fig. 2 ist eine Antriebsvorrichtung 25 in schematischer Form gezeigt. Die Antriebsvorrichtung 25 umfasst ein einziges Antriebsmittel in Form eines Motors 8 und eine erste Omega-förmige Riementransmission 9, bestehend aus einem Rad 10, das mit Hilfe des Motors 8 angetrieben wird, aus zwei Umlenkrollen 11, die auf jeder Seite des Rades 10 angebracht sind und aus einem Zahnriemen 12, der auf dem Rad 10, auf den Umlenkrollen 11 und entlang des Rückens 13 des inneren C-Bogens 3 verläuft. Die Enden des Zahnriemens 12 sind am Rücken 13 des inneren C-Bogens 3 befestigt. Die Antriebsvorrichtung 25 umfasst auch eine zweite Omega-förmige Riementransmission 14 mit einem Rad 15, zwei Umlenkrollen 16 und einen Zahnriemen 17, dessen Enden an dem äußeren C-Bogen 1 befestigt sind. Die Antriebsvorrichtung 25 weist auch eine Kupplungstransmission 18 auf, die die erste und die zweite Riementransmission 9 und 14 verbindet. Die Kupplungstransmission 18 besteht hier aus einem endlosen Antriebsriemen, der an der ersten Riementransmission 9 auf dem Rad 39, das mit dem Motor 8 verbunden ist, verläuft. Der Antriebsriemen verläuft weiterhin über Umlenkrollen 19, die im Anschluss an die erste Riementransmission 9 angeordnet sind bis ein an der zweiten Riementransmission 14 angeordnetes mit dem Rad 15 fest verbundenes Rad 20. Die Riemen 12, 17 und 18 können auch Rollenketten sein.

In Verbindung mit dem Antreiben des inneren C-Bogens 3 in eine orbitale Bewegung, wird das Rad 10 durch den Motor 8, z.B. in die Richtung des Pfeils 21 gedreht. Gleichzeitig werden auch die Räder 39 und 20 und damit auch das Rad 15 mit Hilfe von Umlenkrollen 19 und den Antriebsriemen 18 in Richtung des Pfeils 22 gedreht. Der innere C-Bogen 3 wird nun durch den beschriebenen Aufbau der ersten Riementransmission 9 so angetrieben, dass sie in die Halterung 2 in Richtung des Pfeils 23 verschoben wird, gleichzeitig damit, dass die Halterung 2 durch den beschriebenen Aufbau der zweiten Riementransmission 14 so angetrieben wird, dass sie sich gegenüber den fest im Raum vorhandenen äußeren C-Bogen 1 in dieselbe Richtung wie der innere C-Bogen 3 verschoben wird. Die Übersetzung der Kupplungstransmission wird gewählt, in dem die Größe, d.h. der Durchmesser der Räder 10 und 39 im Verhältnis zum Durchmesser der Räder 20 und 15 bestimmt wird.

Eine Verschiebung des C-Bogens 3 in seine Längsrichtung, eine sogenannte orbitale Bewegung, ist in der Figur mit strichpunktierten Konturen des C-Bogens 1, der Röntgenröhre 4 und des Bildverstärkers 5 gezeigt.

Bei einer Bewegung des C-Bogens in die entgegengesetzte Richtung werden die erwähnten Räder 20, 39 mit Hilfe des Motors 8 in die entgegengesetzte Richtung gedreht.

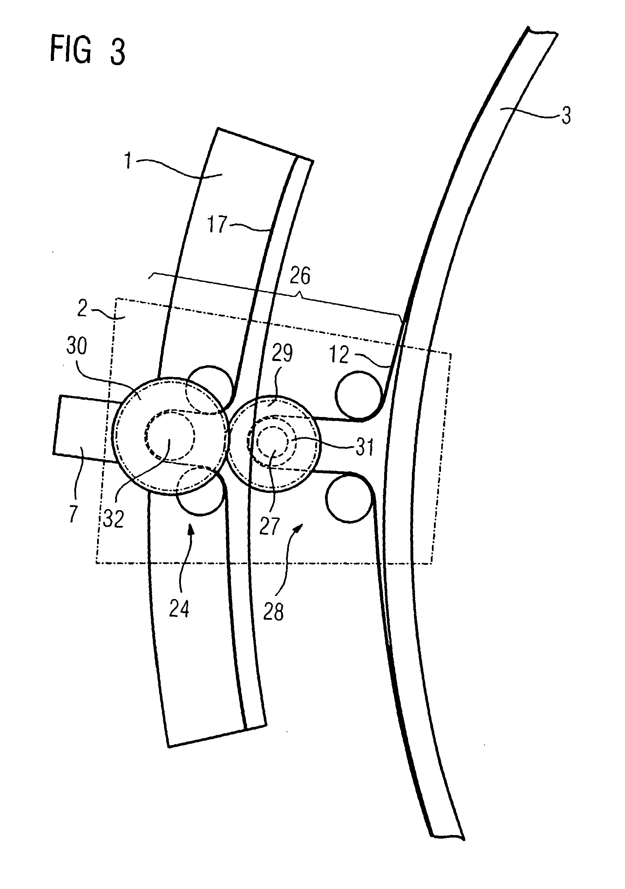

In der Fig. 3 ist eine weitere Antriebsvorrichtung 26 schematisch gezeigt. Die Antriebsvorrichtung 26 weist denselben Aufbau auf wie die in Verbindung mit der Fig. 2 gezeigte Antriebsvorrichtung, d.h. mit einer ersten Riementransmission 28 und einer zweiten Riementransmission 24, wobei die erste Riementransmission 28 mit einem Motor 27 verbunden ist. Statt des in Verbindung mit der FIG. 1 beschriebenen Antriebsriemen 18 besteht in dieser Antriebsvorrichtung 26 die Kupplungstransmission aus einer Zahnradtransmission. Die Zahnradtransmission weist zwei Zahnräder 29, 30 auf, die mit den Rädern 31, 32 für die beiden Riementransmissionen 28, 24 fest verbunden und ineinander eingezahnt sind. Wenn das Rad 31 mit Hilfe des Motors 27 gedreht wird, wird auch das Zahnrad 29 und damit auch das Zahnrad 30 gedreht, wobei eine in Verbindung mit der Antriebsvorrichtung 25 (FIG. 1) ausführlich beschriebene Bewegung des inneren C-Bogens 3 und der Halterung 2 erfolgt. Die Übersetzung der Kupplungstransmission wird vom Durchmesser der Zahnräder 29 und 31 im Verhältnis zum Durchmesser der Räder 30 und 32 bestimmt.

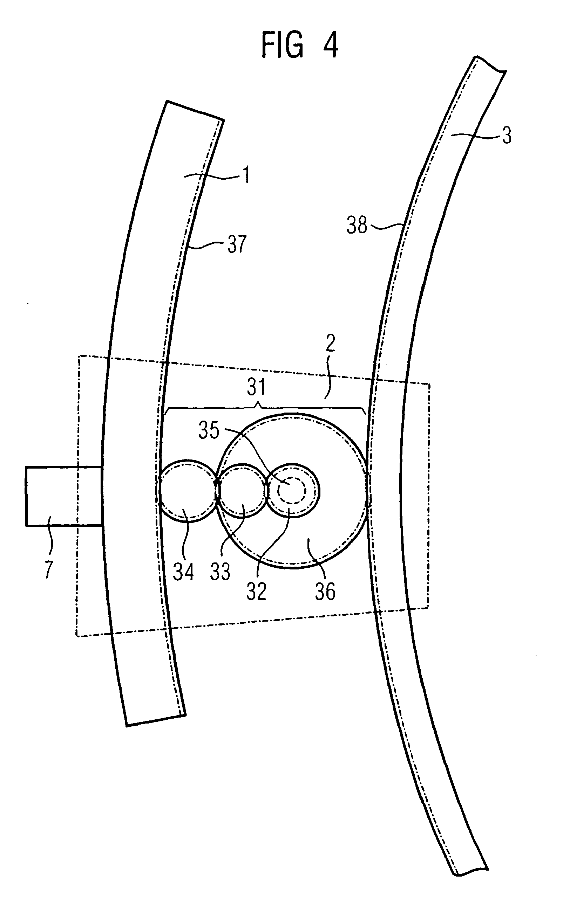

In der FIG. 4 ist eine weitere, im Vergleich zu den bereits beschriebenen Antriebsvorrichtungen 25, 26 vereinfachte Form einer Antriebsvorrichtung 40 schematisch gezeigt. Die Antriebsvorrichtung 40 besteht aus einem Zahnradantrieb, damit eine Übersetzung zwischen dem äußeren und dem inneren C-Bogen 1, 3 geschaffen wird. Der Zahnradantrieb umfasst drei nebeneinander, ineinander gezahnte Zahnräder 32, 33, 34, wobei das Zahnrad 32 mit einem Motor 35 verbunden ist. Ein im Vergleich zu den Zahnrädern 32, 33, 34 größeres Zahnrad 36 ist mit dem Zahnrad 32 fest verbunden. Die Innenseite 37 des äußeren C-Bogens 1 und der Rücken 38 am inneren C-Bogen 3 sind verzahnt. Daher kann das Zahnrad 36 der Antriebsvorrichtung 40 mit Hilfe des Motors 35 den inneren C-Bogen 3 in der Halterung 2 verschieben. Gleichzeitig wird das Zahnrad 34 gezwungen, sich entlang der Innenseite 37 des äußeren C-Bogens 1 in dieselbe Richtung zu verschieben. Die Übersetzung ist hier abhängig vom Größenunterschied zwischen den Zahnrädern 36 und 32.

Durch die Antriebsvorrichtung nach der Erfindung kann das beschriebene Röntgenstativ große orbitale Bewegungen mit einer hohen Geschwindigkeit, einem gleichmäßigen Gang und mit einer hohen Positionierungsgenauigkeit durchführen. All dies ist von großer Bedeutung, da während des Fahrens des inneren C-Bogens gleichzeitig eine größere Anzahl Aufnahmen durchgeführt werden können sollen.

## Patentansprüche

1. Röntgenstativ mit einem äußeren C-Bogen (1), entlang welchem eine Halterung (2) für einen inneren C-Bogen (3) verschiebbar angeordnet ist, wobei der innere C-Bogen (3), der eine Röntgenröhre (4) und einen Bildverstärker (5) trägt, in der Halterung (2) verschiebbar angeordnet ist, und mit einer Antriebsvorrichtung (25, 26, 40) für die Verschiebung der Halterung (2) entlang dem äußeren C-Bogen (1) und für die Verschiebung des inneren C-Bogens (3) entlang der Halterung (2), wobei die Antriebsvorrichtung (25, 26, 40) ein einziges Antriebsmittel (8, 27, 35) aufweist, das den inneren C-Bogen (3) und die Halterung (2) derart gleichzeitig beeinflusst, das sich der innere C-Bogen (3) und die Halterung (2) in dieselbe Richtung bewegen, **dadurch gekennzeichnet,**
**dass** die Antriebsvorrichtung (25, 26, 40) den inneren C-Bogen (3) und die Halterung (2) mit jeweils einer Übersetzung beeinflusst, wobei die Übersetzungen sich gegenseitig derart verhalten wie die Längen der beiden C-Bögen (1, 3).

2. Röntgenstativ nach Anspruch 1, **dadurch gekennzeichnet, dass** die Antriebsvorrichtung (25, 26, 40) in der Halterung (2) angeordnet ist.

3. Röntgenstativ nach Anspruch 1 oder 2, **dadurch gekennzeichnet, dass** die Übersetzung über einen Zahnradantrieb erfolgt.

4. Röntgenstativ nach Anspruch 1 oder 2, **dadurch gekennzeichnet, dass** das Antriebsmittel (8, 27) mit Hilfe einer ersten Riementransmission (9, 28) den inneren C-Bogen (3) verschiebt sowie über eine Kupplungstransmission (18 , 29, 30) ein Kupplungsrad (20, 30) antreibt, das seinerseits über eine zweite Riementransmission (14, 24) die Halterung (2) verschiebt, wobei die Enden des Riemens (12) der ersten Riementransmission (9, 28) an dem inneren C-Bogen (3) und die Enden des Riemens (17) der zweiten Riementransmission (14, 24) an dem äußeren C-Bogen (1) befestigt sind.

5. Röntgenstativ nach Anspruch 4, **dadurch gekennzeichnet, dass** die Kupplungstransmission ein Antriebsriemen (18) ist.

6. Röntgenstativ nach Anspruch 4, **dadurch gekennzeichnet, dass** die Kupplungstransmission eine Rollenkette (18) ist.

7. Röntgenstativ nach Anspruch 4, **dadurch gekennzeichnet, dass** die Kupplungstransmission eine Zahnradtransmission (29, 30) ist.

8. Röntgenstativ nach einem der Ansprüche 1 bis 7, **dadurch gekennzeichnet, dass** der äußere C-Bogen (1) über eine Welle (7) mit einem Stativarm (6) drehbar verbunden ist.

## Claims

1. X-ray stand comprising an outer C arm (1), along which a support (2) for an inner C arm (3) is displaceably mounted, wherein the inner C arm (3), which carries an X-ray tube (4) and an image intensifier (5), is displaceably arranged in the support (2), and comprising a drive device (25, 26, 40) for the displacement of the support (2) along the outer C arm (1) and for the displacement of the inner C arm (3) along the support (2), with the drive device (25, 26, 40) consisting of a single drive means (8, 27, 35) which influences the inner C arm (3) and the support (2) simultaneously in such a manner that the inner C arm (3) and the support (2) move in the same direction
**characterised in that**
the drive device (25, 26, 40) influences the inner C arm (3) and the support (2) with a gear ratio, wherein the gear ratios are in the same ratio to one another as the lengths of the two C arms (1, 3).

2. X-ray stand according to claim 1, **characterised in that** the drive device (25, 26, 40) is arranged in the support (2).

3. X-ray stand according to claim 1 or claim 2, **characterised in that** the gear ratio is effected via a gear drive.

4. X-ray stand according to any one of claims 1 to 3, **characterised in that** the drive means (8, 27) displaces the inner C arm (3) with the aid of a first belt transmission (9, 28) and drives a coupling wheel (20, 30) via a coupling transmission (18, 29, 30), which coupling wheel for its part displaces the support (2) via a second belt transmission (14, 24), whereby the ends of the belt (12) of the first belt transmission (9, 28) are fastened to the inner C arm (3) and the ends of the belt (17) of the second belt transmission (14, 24) are fastened to the outer C arm (1).

5. X-ray stand according to claim 4, **characterised in that** the coupling transmission is a drive belt (18).

6. X-ray stand according to claim 4, **characterised in that** the coupling transmission is a roller chain (18).

7. X-ray stand according to claim 4, **characterised in that** the coupling transmission is a gear transmission (29, 30).

8. X-ray stand according to any one of claims 1 to 7, **characterised in that** the outer C arm (1) is rotatably connected via a shaft (7) to a stand arm (6).

## Revendications

1. Statif de radiographie, comprenant un arceau ( 1 ) extérieur en forme de C, le long duquel une fixation ( 2 ) d'un arceau ( 3 ) intérieur en forme de C est montée coulissante, l'arceau ( 3 ) intérieur en forme de C qui porte un tube ( 4 ) radiogène et un amplificateur ( 5 ) de luminance, étant monté coulissant dans la fixation ( 2 ), et comprenant un dispositif ( 25, 26, 40 ) d'entraînement pour la coulissement de la fixation ( 2 ) le long de l'arceau ( 1 ) extérieur en forme de C et pour le coulissement de l'arceau ( 3 ) intérieur en forme de C le long de la fixation ( 2 ),
le dispositif ( 25, 26, 40 ) d'entraînement ayant un moyen ( 8, 27, 35 ) unique d'entraînement, qui influe en même temps sur l'arceau ( 3 ) intérieur en forme de C et sur la fixation ( 2 ), de manière à ce que l'arceau ( 3 ) intérieur en forme de C et la fixation ( 2 ) se déplacent dans le même sens, **caractérisé**
**en ce que** le dispositif ( 25, 26, 40 ) d'entraînement de l'arceau ( 3 ) intérieur en forme de C et la fixation ( 2 ) sont influencés par, respectivement, une démultiplication, les démultiplications se comportant mutuellement comme les longueurs des deux arceaux ( 1, 3 ) en forme de C.

2. Statif de radiographie suivant la revendication 1, **caractérisé en ce que** le dispositif ( 25, 26, 40 ) d'entraînement est disposé dans la fixation ( 2 ).

3. Statif de radiographie suivant la revendication 1 ou 2, **caractérisé en ce que** la démultiplication s'effectue par un entraînement à roue dentée.

4. Statif de radiographie suivant la revendication 1 ou 2, **caractérisé en ce que** le moyen ( 8, 27 ) d'entraînement déplace l'arceau ( 3 ) intérieur en forme de C au moyen d'une première transmission ( 9, 28 ) à courroie, ainsi qu'entraîne une roue ( 20, 30 ) d'accouplement par une transmission ( 18, 29, 30 ) d'accouplement qui, de son côté, déplace la fixation ( 2 ) par une deuxième transmission ( 14, 24 ) à courroie, les extrémités de la courroie ( 12 ) de la première transmission ( 9, 28 ) à courroie étant fixées à l'arceau ( 3 ) intérieur en forme de C et les extrémités de la courroie ( 17 ) de la deuxième transmission ( 24, 24 ) à courroie étant fixées à l'arceau ( 1 ) extérieur en forme de C.

5. Statif de radiographie suivant la revendication 4, **caractérisé en ce que** la transmission d'accouplement est une courroie ( 18 ) d'entraînement.

6. Statif de radiographie suivant la revendication 4, **caractérisé en ce que** la transmission d'accouplement est une chaîne à rouleau ( 18 ).

7. Statif de radiographie suivant la revendication 4, **caractérisé en ce que** la transmission d'accouplement est une transmission ( 29, 30 ) à roue dentée.

8. Statif de radiographie suivant l'une des revendications 1 à 7, **caractérisé en ce que** l'arceau ( 1 ) extérieur en forme de C est relié avec possibilité de tourner par un arbre ( 7 ) à un bras ( 6 ) du statif.
